# EUROPÄISCHE PATENTANMELDUNG

(11) **EP 2 524 667 A2**
(43) Veröffentlichungstag der Anmeldung: **21.11.2012**
(21) Anmeldenummer: 11186293.4
(22) Anmeldetag: 24.10.2011
(51) Int. Cl.: A61C 1/00

(54) **Medizinisches, insbesondere dentales, Handstück mit einer Temperaturmessvorrichtung**

(30) Priorität: 19.05.2011 EP 11166638
(71) Anmelder: W & H Dentalwerk Bürmoos GmbH, 5111 Bürmoos (AT)
(72) Erfinder: Eder, Karlheinz, 5112 Lamprechtshausen (AT); Pruckner, Christian, 1190 Wien (AT)
(74) Vertreter: Benda, Ralf

(57) **Zusammenfassung**

Medizinisches, insbesondere dentales, Handstück (30), umfassend: Ein Griffteil (31), ein Kupplungsteil (32) zur Verbindung des Handstücks (30) mit zumindest einer Medienquelle und / oder einer Energiequelle, ein Kopfteil (33) mit einer in Bewegung versetzbaren Haltevorrichtung (42) für ein Werkzeug, eine Temperaturmessvorrichtung (34) zur Messung der Temperatur in dem Handstück (30) oder zumindest eines Teils des Handstücks (30), welche ausgebildet ist, ein elektrisches Temperaturmesssignal bereit zu stellen, und eine elektrische Schaltvorrichtung (35), die elektrisch mit der Temperaturmessvorrichtung (34) und mit einer Signalvorrichtung (36, 36A) verbunden ist, um das von der Temperaturmessvorrichtung (34) bereitgestellte Temperaturmesssignal zu empfangen und die Signalvorrichtung (36, 36A) zumindest zwischen einem ersten Signalzustand und einem zweiten Signalzustand, der sich vom ersten Signalzustand unterscheidet, zu schalten, wenn das Temperaturmesssignal einen Grenzwert erreicht oder überschreitet oder unterschreitet.

## Beschreibung

Die vorliegende Erfindung betrifft ein medizinisches, insbesondere dentales, Handstück mit einer Temperaturmessvorrichtung zur Messung der Temperatur des Handstücks oder eines Teils des Handstücks.

Ein derartiges Handstück ist zum Beispiel aus der Patentschrift US 6,786,897 B2 bekannt. Das Handstück ist mit einer lösbaren Kappe oder mit einem Anstrich oder Lack versehen, die einen temperatur-sensitiven oder thermochromen Farbstoff enthalten, der eine Temperaturänderung durch einen Farbwechsel anzeigt.

Der Nachteil dieses Handstücks besteht unter anderem darin, dass der thermochrome Farbstoff die Temperaturänderung nur unpräzise und verzögert wiedergibt und dass die Temperaturmessung und -anzeige ausschließlich an jenen Bauteilen des Handstücks erfolgt, an denen der thermochrome Farbstoff angebracht ist.

Die Anmeldeschrift US 2009/0322541 A1 zeigt ein Handstück, dessen Kopfgehäuse mit einem skelett- oder rippenartigen Gürtel mit einem zentralen Band und davon abstehenden seitlichen Detektionsbändern versehen ist, an dem unter anderem Temperatursensoren vorgesehen sind. Die seitlichen Detektionsbänder übertragen die von unterschiedlichen Wärmequellen abgegebene Wärme mittels Wärmeleitung zu den Temperatursensoren.

Der Nachteil dieses Handstücks besteht unter anderem wiederum in der aufgrund der Wärmeleitung über die Detektionsbänder verzögerten und unpräzisen Temperaturmessung sowie in der durch die Anordnung der Detektionsbänder auf bestimmte Regionen oder Bauteile des Handstücks beschränkten Temperaturmessung.

Der vorliegenden Erfindung liegt daher die Aufgabe zugrunde ein medizinisches, insbesondere dentales, Handstück mit einer alternativen Temperaturmessvorrichtung zur Messung der Temperatur des Handstücks oder eines Teils des Handstücks zu schaffen, welches insbesondere einen oder mehrere der oben genannten Nachteile nicht aufweist.

Gemäß einem Ausführungsbeispiel ist dazu ein medizinisches, insbesondere dentales, Handstück vorgesehen, welches umfasst: Ein Griffteil, ein Kupplungsteil zur Verbindung des Handstücks mit zumindest einer Medienquelle und / oder einer Energiequelle, ein Kopfteil mit einer in Bewegung versetzbaren Haltevorrichtung für ein Werkzeug, eine Temperaturmessvorrichtung zur Messung der Temperatur in dem Handstück oder zumindest eines Teils des Handstücks, welche ausgebildet ist, ein elektrisches Temperaturmesssignal bereit zu stellen, und eine elektrische Schaltvorrichtung, die elektrisch mit der Temperaturmessvorrichtung und mit einer Signalvorrichtung verbunden ist, um das von der Temperaturmessvorrichtung bereitgestellte Temperaturmesssignal zu empfangen und die Signalvorrichtung zumindest zwischen einem ersten Signalzustand und einem zweiten Signalzustand, der sich vom ersten Signalzustand unterscheidet, zu schalten, wenn das Temperaturmesssignal einen Grenzwert erreicht oder überschreitet oder unterschreitet.

Durch die Verwendung einer Temperaturmessvorrichtung, die ein elektrisches Temperaturmesssignal bereit stellt, einer elektrischen Schaltvorrichtung und einer am Handstück vorgesehenen, vorzugsweise ebenfalls elektrisch betreibbaren, Signalvorrichtung wird in vorteilhafter Weise ein Handstück mit einer präzisen und schnellen Temperaturmessung und Temperaturanzeige, welche insbesondere das Erreichen, Über- oder Unterschreiten eines Temperaturgrenzwerts anzeigt, geschaffen.

Die Temperaturmessvorrichtung kann zum Beispiel zumindest ein Thermoelement oder ein Pyrometer oder einen Infrarotsensor oder einen optischen Leiter für elektromagnetische Strahlung, dessen Streuung oder Brechungsindex temperaturabhängig ist, oder einen Temperatursensor, dessen elektrischer oder dynamischer Widerstand temperaturabhängig ist, oder weitere Temperatursensoren, insbesondere alle im Folgenden genannte Temperatursensoren, aufweisen.

Die elektrische Schaltvorrichtung weist vorzugsweise eine Vergleicherschaltung auf, die ausgebildet ist, das von der Temperaturmessvorrichtung bereitgestellte Temperaturmesssignal mit einem vorbestimmten, insbesondere durch einen Anwender einstellbaren, Grenzwert oder Vergleichswert zu vergleichen. Auf Basis dieses Vergleichs steuert die Schaltvorrichtung die Signalvorrichtung an, insbesondere durch Abgabe eines Schaltsignals, um bei Erreichen oder Überschreiten oder Unterschreiten des Grenzwerts die Signalvorrichtung zu aktivieren oder zu deaktivieren. Die elektrische Schaltvorrichtung ist insbesondere als Mikrocontroller oder Microcomputer ausgebildet.

Gemäß einem Ausführungsbeispiel ist / sind die elektrische Schaltvorrichtung und / oder die Temperaturmessvorrichtung und / oder die Signalvorrichtung zur Versorgung mit elektrischer Energie über das Kupplungsteil mit einer außerhalb des Handstücks angeordneten Energiequelle verbindbar, insbesondere mit einer Energieversorgung einer Dentaleinheit oder einer Steuer- und / oder Regeleinheit.

Alternativ ist / sind die elektrische Schaltvorrichtung und / oder die Temperaturmessvorrichtung und / oder die Signalvorrichtung zur Versorgung mit elektrischer Energie mit einer im oder am Handstück vorgesehenen Energiequelle verbunden. Damit wird in vorteilhafter Weise ein in Bezug auf die Temperaturmessung, -verarbeitung und -anzeige von anderen Geräten vollkommen unabhängiges Handstück geschaffen, welches insbesondere problemlos an vorhandene Versorgungs- oder Steuergeräte anschließbar ist. Ein derart vollkommen unabhängiges Handstück wird vorzugsweise durch das Vorsehen eines im oder am Handstück vorgesehenen elektrodynamischen Wandlers (Generators) geschaffen, der durch eine Antriebswelle des Handstücks oder durch ein im Handstück förderbares Fluid betreibbar ist.

Alternativ ist als im oder am Handstück vorgesehene Energiequelle ein Energiespeicher vorgesehen, insbesondere eine Batterie oder ein wiederaufladbaren Akkumulator. Damit kann in vorteilhafter Weise eine von dem Betrieb der Antriebswelle oder von der Förderung des Fluids unabhängige Temperaturmessung erfolgen.

Vorzugsweise umfassen der erste Signalzustand und der zweite Signalzustand ein visuell wahrnehmbares und / oder ein akustisch wahrnehmbares und / oder ein taktil wahrnehmbares Signal, welches insbesondere von der Außenfläche oder Außenhülse des Handstücks und / oder durch die Außenfläche oder Außenhülse unmittelbar auf den Anwender oder in Richtung des Anwenders abgebbar ist. Damit ist eine besonders rasche Übermittlung einer Information über die Temperatur des Handstücks, insbesondere des Erreichens, Über- oder Unterschreitens eines Temperaturgrenzwerts, an den Anwender möglich.

Die Signalvorrichtung weist beispielsweise eine Lichtquelle auf, insbesondere ein optisches Halbleiterelement oder eine LCD-Anzeige. Die Lichtquelle ist vorzugsweise in oder an der Außenfläche oder Außenhülse des Handstücks angeordnet.

Gemäß einem Ausführungsbeispiel ist die Signalvorrichtung, insbesondere die Lichtquelle, ausgebildet, den ersten Signalzustand durch Anzeigen einer ersten Farbe und den zweiten Signalzustand durch Anzeigen einer zweiten Farbe, die sich von der ersten Farbe unterscheidet, darzustellen. Alternativ ist die Signalvorrichtung, insbesondere die Lichtquelle, ausgebildet, den ersten Signalzustand durch Absenden von Licht vom Handstück und den zweiten Signalzustand durch Nicht-Absenden von Licht vom Handstück darzustellen. In beiden Ausführungsbeispielen ist damit eine für den Anwender rasch und deutlich erkennbare Information über die Temperatur des Handstücks, insbesondere des Erreichens, Über- oder Unterschreitens eines Temperaturgrenzwerts, möglich.

Vorzugsweise umfasst die Signalvorrichtung eine Beleuchtungsvorrichtung, die derart am Handstück angeordnet ist oder die dazu ausgebildet ist, dass sie ein in die Haltevorrichtung des Handstücks einsetzbares Werkzeug und / oder die Behandlungsstelle beleuchtet. Diese Beleuchtungsvorrichtung ist insbesondere am Kopfteil des Handstücks oder anschließend an das Kopfteil des Handstücks oder an einem an das Kopfteil angrenzenden Abschnitt des Griffteils angeordnet. In vorteilhafter Weise wird damit nur eine Beleuchtungsvorrichtung oder Lichtquelle benötigt, die sowohl zur Beleuchtung des Werkzeugs und / oder der Behandlungsstelle als auch zur Information über die Temperatur des Handstücks oder eines Teils des Handstücks ausgebildet ist. In vorteilhafter Weise ist es somit für den Anwender, insbesondere während einer Behandlung, nicht nötig sich von der Behandlungsstelle abzuwenden, wenn er eine Information über die Temperatur des Handstücks wünscht.

Gemäß einem Ausführungsbeispiel ist die Signalvorrichtung, insbesondere die Lichtquelle, am Griffteil oder am Kupplungsteil des Handstücks vorgesehen, insbesondere an einer Seite des Griffteils oder des Kupplungsteils, die von einer Werkzeugaufnahmeöffnung des Handstücks abgewandt oder dieser gegenüber liegt, wodurch eine besonders gute Sichtbarkeit der Signalvorrichtung für den Anwender erreicht wird.

Um eine möglichst rasche und exakte Temperaturmessung zu ermöglichen, ist die Temperaturmessvorrichtung bevorzugt nahe zumindest einer Wärmequelle angeordnet, vorzugsweise im oder anschließend an das Kopfteil des Handstücks und / oder an oder nahe an einem bewegten Bauteil des Handstücks, zum Beispiel an einem Lager, einer Welle, einer Haltevorrichtung für ein mit dem Handstück verbindbaren Werkzeug oder einer Lösevorrichtung für ein mit dem Handstück verbindbaren Werkzeug. Als Wärmequelle, deren Temperatur mittels der Temperaturmessvorrichtung bestimmbar ist, kann jedoch auch ein im Handstück vorgesehener Antrieb, zum Beispiel ein Elektromotor, oder eine andere, zum Beispiel mit elektrischer Energie betreibbare, Vorrichtung ausgebildet sein.

Vorzugsweise ist die Temperaturmessvorrichtung ausgebildet, die Temperatur des Handstücks oder eines Teils des Handstücks berührungslos durch Detektion von elektromagnetischer Strahlung, insbesondere von Wärmestrahlung, zu messen und / oder die Temperaturmessvorrichtung ist ausgebildet, die Temperatur eines durch zumindest eine Wärmequelle erwärmbaren Innenraums des Handstücks zu messen. In vorteilhafter Weise kann die Temperaturmessung damit sehr rasch und unabhängig, insbesondere räumlich getrennt, von der Wärmequelle erfolgen, so wie dies im Folgenden noch im Detail beschrieben ist.

Vorzugsweise weist das Handstück zumindest zwei lösbar miteinander verbundene Teile auf, wobei die elektrische Schaltvorrichtung und / oder die Temperaturmessvorrichtung und / oder die Signalvorrichtung und / oder die im oder am Handstück vorgesehenen Energiequelle in unterschiedlichen Teilen angeordnet sind. Die lösbar miteinander verbundene Teile sind zum Beispiel durch das Griffteil und ein davon trennbares Kopfteil oder durch ein Griffteil und davon lösbares Kupplungsteil zur Verbindung des Handstücks mit zumindest einer Medienquelle und / oder einer Energiequelle gebildet.

Gemäß einem alternativen Ausführungsbeispiel ist ein medizinisches, insbesondere dentales, Handstück vorgesehen, welches umfasst: Eine Außenhülse, eine Antriebsvorrichtung zum in Bewegung Versetzen eines mit dem Handstück verbindbaren Werkzeugs und eine Temperaturmessvorrichtung zur Messung der Temperatur des Handstücks oder eines Teils des Handstücks, wobei die Temperaturmessvorrichtung ausgebildet ist, die Temperatur des Handstücks oder eines Teils des Handstücks, zum Beispiel eines oder mehrere Bauteile des Handstücks oder eines Innenraums des Handstücks, berührungslos durch Detektion von elektromagnetischer Strahlung, insbesondere von Wärmestrahlung, zu messen.

Der Vorteil einer Temperaturmessvorrichtung, welche die Temperatur berührungslos durch Detektion von elektromagnetischer Strahlung misst, besteht unter anderem darin, dass die Temperaturmessung sehr rasch und unabhängig, insbesondere räumlich getrennt, von der Wärmequelle erfolgt. Aufgrund der räumlichen Trennung ist vorzugsweise eine Temperaturmessung beweglicher oder bewegter Bauteile des Handstücks möglich, ohne diese zu kontaktieren, wodurch insbesondere eine Abnützung des Temperatursensors vermieden wird. Durch die Detektion von elektromagnetischer Strahlung wird die Messung der Temperatur auch unabhängig von äußeren Einflüssen, zum Beispiel von Konvektion.

Gemäß einem Ausführungsbeispiel ist die Temperaturmessvorrichtung angeordnet und / oder ausgebildet, elektromagnetische Strahlung mehrerer in oder an der Außenhülse des Handstücks angeordneter Wärmequellen und / oder der Außenhülse zu empfangen, um insbesondere einen gemittelten Temperaturwert der mehreren Wärmequellen und / oder der Außenhülse zu bestimmen. Vorzugsweise ist die Temperaturmessvorrichtung, welche die Temperatur berührungslos durch Detektion von elektromagnetischer Strahlung misst, derart angeordnet und / oder ausgebildet, dass sie einen gemittelten Temperaturwert oder Durchschnittstemperaturwert ermittelt, insbesondere einen gemittelten Temperaturwert oder Durchschnittstemperaturwert mehrerer Bauteile und / oder Wärmequellen des Handstücks und / oder eines die Temperaturmessvorrichtung umgebenden Raumvolumens, zum Beispiel eines Teilvolumens des Handstücks, insbesondere des Handstückkopfs des Handstücks, in welchem ein Werkzeughalter für das mit dem Handstück verbindbare Werkzeug vorgesehen ist. Das im Vorstehenden genannte Raum- oder Teilvolumen, dessen gemittelte Temperatur oder Durchschnittstemperatur messbar ist, ist insbesondere größer als das Volumen der Temperaturmessvorrichtung oder des Sensors der Temperaturmessvorrichtung, vorzugsweise mehrfach größer als das Volumen der Temperaturmessvorrichtung oder des Sensors der Temperaturmessvorrichtung.

Eine Temperaturmessvorrichtung, welche elektromagnetische Strahlung mehrerer Wärmequellen empfängt oder welche einen gemittelten Temperaturwert oder Durchschnittstemperaturwert ermittelt, ist zum Beispiel zwischen den mehreren Wärmequellen und / oder Bauteilen angeordnet und / oder weist mehrere elektromagnetische Strahlung empfangende Seitenflächen auf, insbesondere in unterschiedliche Richtungen weisende, elektromagnetische Strahlung empfangende Seitenflächen, um elektromagnetische Strahlung oder Wärmestrahlung aus unterschiedlichen Richtungen zu empfangen.

Gemäß einem Ausführungsbeispiel sind mehrere Wärmequellen am oder im Handstück vorhanden, wobei insbesondere eine Wärmequelle das Handstück oder Teile des Handstücks stärker erwärmt als eine andere Wärmequelle oder wobei insbesondere eine Wärmequelle mehr Wärme oder Wärmestrahlung emittiert als eine andere Wärmequelle. Beispielsweise sind im Handstück oder im Handstückkopf zwei Lager, insbesondere Wälzlager, vorgesehen, wobei ein erstes Lager aufgrund eines Gebrechens oder aufgrund einer Verschmutzung wärmer wird als ein zweites Lager. Vorzugsweise ist die Temperaturmessvorrichtung, welche die Temperatur berührungslos durch Detektion von elektromagnetischer Strahlung misst, derart angeordnet und / oder ausgebildet, dass sie einen Temperaturwert misst, insbesondere eine Temperatur des Handstücks oder eines Teils des Handstücks, die von den mehreren Wärmequellen beeinflusst wird. Bevorzugt ist die Temperaturmessvorrichtung derart angeordnet und / oder ausgebildet, dass sie einen gemittelten Temperaturwert oder Durchschnittstemperaturwert des Handstücks oder eines Teils des Handstücks oder eines Innenraums des Handstücks misst, die aus den Wärmeemissionen der mehreren Wärmequellen resultiert oder die sich aus den Wärmeemissionen der mehreren Wärmequellen zusammensetzt. In vorteilhafter Weise misst die Temperaturmessvorrichtung somit nicht ausschließlich oder nicht vorwiegend die Temperatur eines bestimmten Bauteils.

Vorzugsweise ist die Temperaturmessvorrichtung, welche die Temperatur berührungslos durch Detektion von elektromagnetischer Strahlung misst, derart angeordnet und / oder ausgebildet, dass sie einen Temperaturwert misst, insbesondere einen Temperaturwert des Handstücks oder eines Teils des Handstücks oder eines Innenraums des Handstücks, bevorzugt einen gemittelten Temperaturwert oder Durchschnittstemperaturwert, der von einem oder mehreren Bauteilen des Handstücks beeinflusst wird, die nicht als (primäre oder unmittelbare) Wärmequelle ausgebildet sind, sondern durch eine im Handstück vorgesehene Wärmequelle erwärmbar sind, zum Beispiel unbewegliche Bauteile, die mit einer Wärmequelle verbunden sind und von dieser erwärmt werden, zum Beispiel durch Wärmeleitung oder Konvektion. Besonders bevorzugt ist die Temperaturmessvorrichtung, welche die Temperatur berührungslos durch Detektion von elektromagnetischer Strahlung misst, derart angeordnet und / oder ausgebildet, dass sie einen Temperaturwert misst, bevorzugt einen gemittelten Temperaturwert oder Durchschnittstemperaturwert, der von zumindest einer Wärmequelle und zumindest einem Bauteil des Handstücks beeinflusst wird, das nicht direkt als Wärmequelle ausgebildet ist, sondern durch eine im Handstück vorgesehene Wärmequelle erwärmbar ist.

Selbstverständlich können die im Vorstehenden genannten Wärmequellen auch andere Bauteile des Handstücks als die genannten Lager umfassen, insbesondere jegliche relativ zueinander bewegte Bauteile des Handstücks, vorzugsweise bewegbare und stillstehende, einander kontaktierende Bauteile, zum Beispiel einen Werkzeughalter und eine Werkzeuglösevorrichtung des Handstücks oder Teile des Werkzeughalters und der Werkzeuglösevorrichtung oder einen Rotor eines Motors oder eines Generators oder die Außenhülse des Handstücks und ein relativ dazu bewegliches Bauteil des Handstücks. Eine Wärmequelle kann auch durch ein mit elektrischer Energie versorgbares Bauteil, wie zum Beispiel eine Beleuchtungsvorrichtung, einen Elektromotor, insbesondere dessen Spulen, eine elektrische oder elektronische Schaltung, einen Sensor oder Detektor etc. gebildet sein. Diese Aufzählung der möglichen Wärmequellen ist beispielhaft und nicht vollständig und trifft in entsprechender Weise auf alle weiteren beschriebenen Ausführungsbeispiele zu.

Vorzugsweise ist die Temperaturmessvorrichtung, die ausgebildet ist, die Temperatur des Handstücks oder eines Teils des Handstücks berührungslos durch Detektion von elektromagnetischer Strahlung zu messen, im Handstück oder im Inneren der Außenhülse des Handstücks angeordnet, wodurch eine besonders schnelle, unmittelbare und präzise Messung gewährleistet wird.

Gemäß einem Ausführungsbeispiel weist die zumindest eine in der Außenhülse des Handstücks angeordnete Wärmequelle eine elektromagnetische Strahlung emittierende Oberfläche auf, welche räumlich von einer die elektromagnetische Strahlung der Wärmequelle empfangenden Oberfläche der Temperaturmessvorrichtung beabstandet ist. Durch diese räumliche Trennung ist es in vorteilhafter Weise insbesondere möglich, die Temperaturen mehrerer Wärmequellen und / oder Bauteile, die mit einer Wärmequelle verbunden sind und von dieser erwärmt werden, zu messen und / oder einen gemittelten Temperaturwert oder Durchschnittstemperaturwert des Handstücks oder eines Teils des Handstücks oder eines Innenraums des Handstücks zu messen, der aus den Wärmeemissionen der Wärmequellen und / oder der Bauteile resultiert.

Gemäß weiteren Ausführungsbeispielen umfasst die Temperaturmessvorrichtung, die ausgebildet ist, die Temperatur des Handstücks oder eines Teils des Handstücks berührungslos durch Detektion von elektromagnetischer Strahlung zu messen, ein Pyrometer oder einen Infrarotsensor, die insbesondere ausgebildet sind, elektromagnetische Strahlung in einem Bereich von etwa 5 µm bis etwa 20 µm zu detektieren. Alternativ weist die Temperaturmessvorrichtung einen optischen Leiter für elektromagnetische Strahlung auf, insbesondere einen Faserstab, dessen Streuung oder Brechungsindex aufgrund von Temperaturänderungen variiert.

Vorzugsweise kann am Pyrometer oder Infrarotsensor ein Strahlungsleiter vorgesehen sein, der ausgebildet ist, Wärmestrahlung, insbesondere Infrarot-Strahlung, zu dem Pyrometer oder Infrarotsensor zu leiten. Dies ist insbesondere von Vorteil, wenn das Pyrometer oder der Infrarotsensor nicht an dem Ort positionierbar ist, an dem die Temperaturmessung gewünscht ist.

Gemäß einem alternativen Ausführungsbeispiel ist ein medizinisches, insbesondere dentales, Handstück vorgesehen, das umfasst: Eine hohle Außenhülse mit einem Innenraum, eine Antriebsvorrichtung zum in Bewegung Versetzen eines mit dem Handstück verbindbaren Werkzeugs, zumindest eine in der Außenhülse des Handstücks angeordnete Wärmequelle, welche durch Abgabe von Wärme in den Innenraum den Innenraum erwärmt, und eine Temperaturmessvorrichtung, die ausgebildet ist, die Temperatur des durch die zumindest eine Wärmequelle erwärmbaren Innenraums des Handstücks zu messen.

Ein Vorteil dieses Handstücks besteht darin, dass die Temperaturmessvorrichtung somit nicht ausschließlich oder nicht vorwiegend die Temperatur eines bestimmten Bauteils misst, sondern dass vorzugsweise, insbesondere bei Vorhandensein mehrerer Wärmequellen, mittels der Temperaturmessvorrichtung ein gemittelter Temperaturwert oder Durchschnittstemperaturwert ermittelbar ist. Somit können in vorteilhafter Weise insbesondere mehrere Wärmequellen durch eine geringere Anzahl von Temperaturmessvorrichtung überwacht werden, vorzugsweise mehrere Wärmequellen durch eine einzige Temperaturmessvorrichtung überwacht werden. Die mehreren Wärmequellen geben zumindest einen Teil ihrer Wärme in den Innenraum des Handstücks ab, bevorzugt in ein Teilvolumen des Handstücks, insbesondere in den Handstückkopf des Handstücks, in welchem ein Werkzeughalter für das mit dem Handstück verbindbare Werkzeug vorgesehen ist. Das im Vorstehenden genannte Teilvolumen bzw. der Innenraum, dessen gemittelte Temperatur oder Durchschnittstemperatur messbar ist, ist insbesondere größer als das Volumen der Temperaturmessvorrichtung oder des Sensors der Temperaturmessvorrichtung, vorzugsweise mehrfach größer als das Volumen der Temperaturmessvorrichtung oder des Sensors der Temperaturmessvorrichtung.

Der Innenraum des Handstücks ist vorzugsweise durch ein Fluid, insbesondere Luft, enthaltendes Volumen gebildet, welches Bauteile des Handstücks voneinander trennt, wobei in dem Volumen die Temperaturmessvorrichtung vorgesehen ist. In dieses Volumen gibt die zumindest eine Wärmequelle ihre Wärme ab. Alternativ oder zusätzlich ist die Temperaturmessvorrichtung räumlich von der zumindest einen Wärmequelle beabstandet. Beide Maßnahmen erhöhen die Unabhängigkeit der Temperaturmessung von der zumindest einen Wärmequelle oder erleichtern die Bestimmung eines gemittelten Temperaturwerts oder Durchschnittstemperaturwerts.

Die Temperaturmessvorrichtung, die ausgebildet ist, die Temperatur des durch die zumindest eine Wärmequelle erwärmbaren Innenraums des Handstücks zu messen, umfasst zum Beispiel ein Pyrometer oder einen Infrarotsensor, die insbesondere ausgebildet sind, elektromagnetische Strahlung in einem Bereich von etwa 5 µm bis etwa 20 µm zu detektieren, oder einen optischen Leiter für elektromagnetische Strahlung, insbesondere einen Faserstab, dessen Streuung oder Brechungsindex aufgrund von Temperaturänderungen variiert, oder eine elektrische Temperaturmessvorrichtung, zum Beispiel ein Thermoelement oder eine Temperaturmessvorrichtung, die ein Material umfasst, dessen elektrischer Widerstand oder dynamischer Widerstand in Abhängigkeit der Temperatur variabel ist.

Gemäß einem weiteren, alternativen Ausführungsbeispiel ist ein medizinisches, insbesondere dentales, Handstück vorgesehen, das umfasst: Eine Außenhülse, eine Antriebsvorrichtung zum in Bewegung Versetzen eines mit dem Handstück verbindbaren Werkzeugs und eine Temperaturmessvorrichtung zur Messung der Temperatur des Handstücks oder eines Teils des Handstücks, wobei die Temperaturmessvorrichtung als elektrische Temperaturmessvorrichtung ausgebildet ist.

Ein Vorteil einer elektrischen Temperaturmessvorrichtung besteht unter anderem in der präzisen und raschen Ermittlung des Temperarturwerts.

Gemäß einem ersten Ausführungsbeispiel weist die elektrische Temperaturmessvorrichtung zumindest ein Thermoelement auf, welches ausgebildet ist, zur Messung der Temperatur ein elektrisches Thermospannungssignal zur Verfügung zu stellen. Der Vorteil eines Thermoelements liegt insbesondere in seinem einfachen Aufbau und seiner Robustheit, insbesondere gegenüber externen Einflüssen, die zum Beispiel während der Reinigung oder Sterilisation des Handstücks auftreten. Vorzugsweise ist eine dem Thermoelement zugeordnete Auswerteeinheit vorgesehen, die ausgebildet ist, aus dem elektrischen Thermospannungssignal des Thermoelements einen Temperaturwert zu ermitteln.

Der Begriff Thermoelement umfasst eine Vorrichtung, die durch Thermoelektrizität Wärme in elektrische Energie wandelt. Insbesondere weist ein Thermoelement zwei unterschiedliche und an einem Ende miteinander verbundene, elektrisch leitende Materialien, insbesondere Metalle oder Metalldrähte auf, um damit Temperaturmessungen durchführen zu können. Ein Thermoelement weist somit bevorzugt einen bimetallischen Thermokontakt auf. Eine Temperaturdifferenz an den Enden der Metalle oder Metalldrähte erzeugt aufgrund des Seebeck-Effekts innerhalb der beiden Metalle eine elektrische Ladungstrennung oder eine elektrische Thermospannung. Diese elektrische Potentialdifferenz ist annähernd proportional zur Temperaturdifferenz an den Enden der Metalle. Um die absolute Temperatur an den Metallen oder Drahtenden bestimmen zu können, müssen weitere Maßnahmen ergriffen werden, zum Beispiel die Messung der Umgebungstemperatur, zu der die Temperaturdifferenz an den Enden der Metalle addiert wird. Da der Aufbau und die Funktion eines Thermoelements bekannt sind, wird darauf nicht weiter eingegangen.

Das zumindest eine Thermoelement umfasst vorzugsweise zwei elektrisch leitende Materialien, insbesondere ein Metall oder einen Metalldraht, zum Beispiel Stahl, Kupfer, eine Kupfer-Nickel-Legierung (Konstantan) oder andere Legierungen, welche zum Beispiel Nickel, Chrom, Eisen oder Kupfer enthalten.

Vorzugsweise ist das zumindest eine Thermoelement in einem Innenraum des Handstücks angeordnet, wobei der Innenraum durch ein Fluid, insbesondere Luft, enthaltendes Volumen gebildet ist, welches Bauteile des Handstücks voneinander trennt. In vorteilhafter Weise misst das zumindest eine Thermoelement damit insbesondere die Temperatur des Innentraums und / oder die (gemittelte) Temperatur mehrerer Wärmequellen, welche Wärme in den Innenraum abgeben, und / oder einen gemittelten Temperaturwert oder Durchschnittstemperaturwert, insbesondere einen gemittelten Temperaturwert oder Durchschnittstemperaturwert mehrerer Bauteile und / oder Wärmequellen des Handstücks und / oder eines die Temperaturmessvorrichtung umgebenden Raumvolumens, zum Beispiel eines Teilvolumens des Handstücks.

Alternativ ist das zumindest eine Thermoelement an einem Bauteil des Handstücks vorgesehen, insbesondere an einer Wärmequelle oder an einem Wärme abgebenden Bauteil, wobei bevorzugt das Bauteil des Handstücks, an dem das Thermoelement vorgesehen ist, ein Material aufweist, welches Teil des Thermoelements oder der Thermoelementverbindung ist. Durch das Vorsehen des Thermoelements an einem Bauteil des Handstücks kann in vorteilhafter Weise insbesondere die Temperatur dieses Bauteils bestimmt werden. Durch das Verwenden eines Materials des Bauteils, an dem das Thermoelement angeordnet ist, als Teil des Thermoelements oder der Thermoelementverbindung kann in vorteilhafter Weise die Baugröße des Thermoelements verringert werden und kann, insbesondere wenn es sich um ein größeres Bauteil handelt, zum Beispiel um die Außenhülse des Handstücks, die Kontaktierung des Thermoelements, die das Thermospannungssignal ableitet, entfernt von der eigentlichen Thermoelementverbindung (d.h. dem Kontaktpunkt oder -bereich der beiden Materialien des Thermoelements) angeordnet sein, wodurch die Anordnung des Thermoelements bzw. der Kontakte und Leitungen zur Ableitung der Thermospannungssignal im beengten Inneren des Handstücks vereinfacht wird. Bevorzugt ist das Bauteil des Handstücks, an dem das Thermoelement vorgesehen ist und das ein Material aufweist, welches Teil des Thermoelements oder der Thermoelementverbindung ist, durch eine Außenhülse des Handstücks oder durch ein mit der Außenhülse des Handstücks (elektrisch leitend) verbundenes Bauteil des Handstücks, zum Beispiel einem Lager zur Lagerung eines Werkzeughalters für das mit dem Handstück verbindbare Werkzeug oder einem Kontaktring, gebildet.

Gemäß einem zweiten Ausführungsbeispiel umfasst die elektrische Temperaturmessvorrichtung ein Material, dessen elektrischer Widerstand oder dynamischer Widerstand in Abhängigkeit der Temperatur variabel ist. Das Material umfasst zum Beispiel einen Ohmschen Widerstand, einen elektrischen Leiter, insbesondere ein Metall, eine Keramik (ein gesintertes Metalloxid) oder einen dotierten Halbleiter. Bevorzugt ist zumindest eines dieser Materialien Teil eines Sensors der elektrischen Temperaturmessvorrichtung. Je nach Ausführung ist ein derartiger Sensor als aktiver Sensor, der zum Bestimmen der Temperatur eine Strom- oder Spannungsversorgung benötigt, oder als passiver Sensor ausgebildet, der zum Bestimmen der Temperatur keine Strom- oder Spannungsversorgung benötigt.

Der Begriff Handstück umfasst insbesondere mit einer Hand greifbare medizinische oder dentale Geräte, zum Beispiel gerade, pistolenförmige, gewinkelte oder gebogene Handgriffe, die im Dentalbereich oftmals als Winkelstücke bezeichnet werden, Teile von Handstücken oder Handgriffen, insbesondere einen Kopfabschnitt, der zum Beispiel mit einem davon lösbaren Griffabschnitten verbindbar ist, sowie Kupplungen, Adapter, Verbindungsstücke und Antriebseinheiten, zum Beispiel elektrische oder pneumatische Motoren. Unter der Bezeichnung Handstück werden des Weiteren sowohl schnurlose Handstück verstanden, insbesondere mit einer austauschbaren oder aufladbaren Energiequelle, als auch Handstück, die eine Versorgungsleitung und eine damit verbundene Steuer-, Regel- und / oder Versorgungseinheit umfassen. Gemäß einem bevorzugten Ausführungsbeispiel, welches auf alle genannten Ausführungsformen und -beispiele anwendbar ist, weist das Handstück einen Handstückkopf auf, in welchem ein Werkzeughalter für das mit dem Handstück verbindbare Werkzeug und die Temperaturmessvorrichtung, insbesondere der Temperatursensor der Temperaturmessvorrichtung, vorgesehen sind.

Die Erfindung wird nachfolgend anhand bevorzugter Ausführungsbeispiele und Bezug nehmend auf die beigefügten Zeichnungen erläutert:
Figur 1 zeigt eine Außenansicht eines Ausführungsbeispiels eines erfindungsgemäßen Handstücks mit einer Temperaturmessvorrichtung zur Messung der Temperatur des Handstücks oder eines Teils des Handstücks.
Figur 2 zeigt ein Ausführungsbeispiel eines Handstückkopfs mit einer Temperaturmessvorrichtung zur Messung der Temperatur des Handstücks oder eines Teils des Handstücks, wobei die Temperaturmessvorrichtung ein Pyrometer oder einen Infrarotsensor umfasst.
Die Figuren 3 und 4 zeigen zwei Ausführungsbeispiele von Handstückköpfen mit je einer Temperaturmessvorrichtung zur Messung der Temperatur des Handstücks oder eines Teils des Handstücks, wobei die Temperaturmessvorrichtungen jeweils einen optischen Leiter für elektromagnetische Strahlung umfasst, dessen Streuung oder Brechungsindex aufgrund von Temperaturänderungen variiert.
Die Figuren 5 - 9 zeigen Ausführungsbeispiele von Handstückköpfen mit je einer Temperaturmessvorrichtung zur Messung der Temperatur des Handstücks oder eines Teils des Handstücks, wobei die Temperaturmessvorrichtungen jeweils ein Thermoelement umfassen.
Figur 10 zeigt ein Ausführungsbeispiel eines Handstückkopfs mit einer Temperaturmessvorrichtung zur Messung der Temperatur des Handstücks oder eines Teils des Handstücks, wobei die Temperaturmessvorrichtung zwei Thermoelemente umfasst.
Die Figuren 11 - 13 zeigen Ausführungsbeispiele von Handstückköpfen mit je einer Temperaturmessvorrichtung zur Messung der Temperatur des Handstücks oder eines Teils des Handstücks, wobei die elektrische Temperaturmessvorrichtung ein Material umfasst, dessen elektrischer Widerstand oder dynamischer Widerstand in Abhängigkeit der Temperatur variabel ist.
Die Figuren 14 - 16 zeigen Ausführungsbeispiele medizinischer, insbesondere dentaler, Handstücke mit einer elektrischen Schaltvorrichtung, die elektrisch mit der Temperaturmessvorrichtung und mit einer Signalvorrichtung verbunden ist.

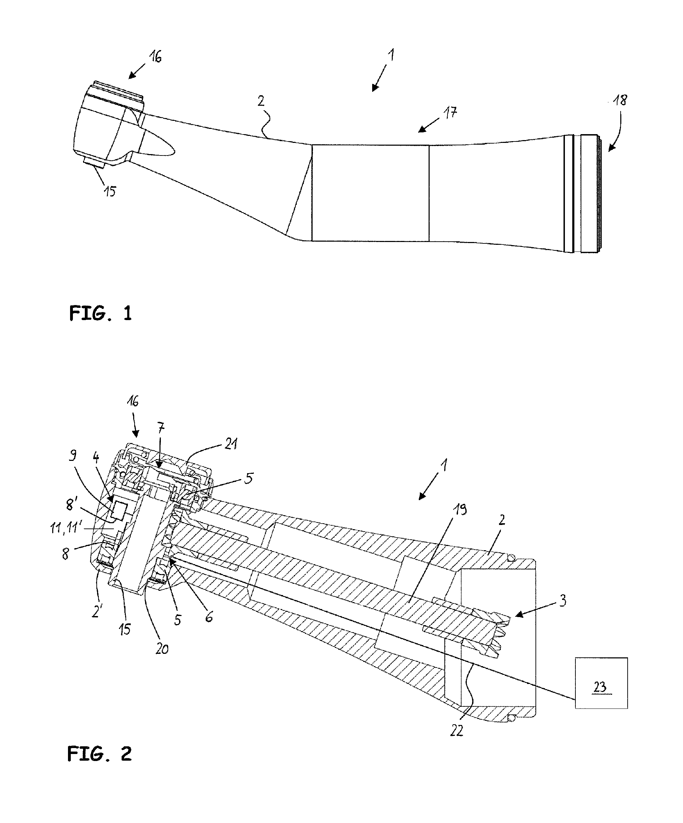

Die Figur 1 zeigt eine Außenansicht eines gebogenen Handstücks 1, welches oftmals als Winkelstück bezeichnet wird, mit seiner Außenhülse 2. Das Handstück 1 weist einen, insbesondere gebogenen, Griffteil 17 und einen Kopfteil 16 auf, in welchem ein Werkzeughalter 15 zum lösbaren Verbinden oder Aufnehmen eines mit dem Handstück 1 verbindbaren Werkzeugs angeordnet ist. Der Werkzeughalter 15 ist vorzugsweise beweglich im Handstück 1 angeordnet, zum Beispiel drehbar, wobei die Drehbarkeit insbesondere durch Lager 5, vorzugsweise Wälz- oder Kugellager, gewährleistet ist (siehe Figur 2).

An dem dem Kopfteil 16 gegenüber liegenden Ende des Handstücks 1 ist eine Kupplungs-oder Anschlussvorrichtung 18 vorgesehen, welche ausgebildet ist, das Handstück 1 mit einer oder mehreren Fluidquellen, zum Beispiel einer Flüssigkeitsquelle, insbesondere Wasser, oder einer Gasquelle, insbesondere Druckluft, zu verbinden. Vorzugsweise ist das Handstück 1 über die Anschlussvorrichtung 18 auch mit einer elektrischen Spannungsquelle und / oder einer Antriebsvorrichtung, zum Beispiel einen Elektromotor oder einem pneumatisch betreibbaren Motor, verbindbar. Wahlweise sind weitere elektrische Leitungen oder Kontakte zur Daten- und / oder Signalübertragung an der Kupplungsvorrichtung 18 vorgesehen.

Durch das Handstück 1 bzw. die Außenhülse 2 erstrecken sich eine oder mehrere Fluidleitungen von der Anschlussvorrichtung 18 in Richtung des Kopfteils 16 oder bis in den Kopfteil 16. Gegebenenfalls weist das Handstück 1 auch eine oder mehrere Wellen 19 auf, vorzugsweise mittels Zahnrädern oder Getrieben 6 verbunden, die als Antriebsvorrichtung 3 dienen und eine Antriebsbewegung auf den Werkzeughalter 15 übertragen (siehe Figur 2).

Die Figur 2 zeigt einen Schnitt durch einen Teil des Griffteils 17 und insbesondere durch den Kopfteil 16 des Handstücks 1. Der Kopfteil 16 weist eine hohle Kopfhülse 2' auf, die einen Innenraum 11 bildet. Der Innenraum 11 ist insbesondere als Fluid, insbesondere Luft, enthaltendes Volumen 11' ausgebildet, in dem unterschiedliche Bauteile des Kopfteils aufgenommen sind, zum Beispiel in Bezug auf die Hülse 2, 2' stationäre Bauteile oder in Bezug auf die Hülse 2, 2' bewegliche Bauteile, insbesondere die Lager 5, der Werkzeughalter 15, zumindest Teile eines Getriebes 6 oder von Zahnrädern, welche die Antriebsbewegung der Antriebsvorrichtung 3 von der Welle 19 auf den Werkzeughalter 15 übertragen, zumindest Teile eine Vorrichtung 7 zum Lösen des Werkzeugs aus dem Werkzeughalter oder weitere Bauteile wie Federelemente, Stützelemente, etc. An einer Seite des Kopfteils 16 ist des Weiteren eine Öffnung 20 vorgesehen, durch welche das Werkzeug in den Kopfteil 16 oder in den Werkzeughalter 15 einsetzbar ist bzw. daraus entnehmbar ist. An der Seite des Kopfteils 16, die der Öffnung 20 gegenüber liegt, ist ein Betätigungselement 21 zum Betätigen der Werkzeuglösevorrichtung 7 vorgesehen, insbesondere ein Druckelement oder Druckdeckel.

Zumindest einige der im Vorstehenden genannten Bauteile oder Teile davon können als Wärmequellen ausgebildet sein, zum Beispiel die Lager 5, das Getriebe 6 oder die Werkzeuglösevorrichtung 7. Selbstverständlich können jedoch auch andere Bauteile des Handstücks Wärmequellen bilden, insbesondere ein elektromagnetische Strahlung emittierendes Bauteil, zum Beispiel eine Beleuchtungsvorrichtung, ein Heizelement für Medien, ein elektrisches oder elektronisches Bauteil, etc. Gemeinsam ist allen diesen Wärmequellen, dass sie Wärme in das Innere des Handstücks 1 und / oder an die Außenhülse 2, 2' des Handstücks abgeben. Um die die Temperatur des Handstücks 1 oder eines Teils des Handstücks 1, vorzugsweise der Außenhülse 2, 2', oder des Innenraums 11 des Handstücks 1 zu bestimmen und insbesondere eine zu starke Erwärmung zu verhindern, ist deshalb am oder im Handstück 1, insbesondere am oder im Kopfteil 16, eine Temperaturmessvorrichtung 4 vorgesehen.

Über eine elektrische Leitung oder eine Signalleitung 22 ist die Temperaturmessvorrichtung 4 mit einer Einheit 23 verbunden, die wahlweise als Auswerteeinheit zum Auswerten der Messsignale der Temperaturmessvorrichtung 4 und / oder als Regel- oder Steuereinheit zum temperaturabhängigen Regeln oder Steuern des Betriebs des Handstücks 1, zum Beispiel zum Unterbrechen des Betriebs des Handstücks 1 bei Erreichen oder Überschreiten eines vorbestimmten Temperaturgrenzwerts, und / oder als Anzeigeeinheit zum Anzeigen des gemessenen oder bestimmten Temperaturwerts oder eines Temperaturgrenzwerts ausgebildet ist. Vorzugsweise sind zumindest Teile der Einheit 23 am oder im Handstück 23 vorgesehen. Gemäß einem besonders bevorzugten Ausführungsbeispiel ist die gesamte Temperaturmessvorrichtung 4 einschließlich der Leitung 22 und der Einheit 23 im oder am Handstück 1 angeordnet, wodurch insbesondere ein in Bezug auf die Temperaturmessung, -auswertung und die davon abhängige Steuerung oder Regelung unabhängiges Handstück 1 entsteht.

Die im Vorstehenden unter Bezug auf die Figuren 1 und 2 beschriebenen Merkmale gelten für alle in den Figuren 2 - 13 beschriebenen Ausführungsbeispiele. Im Folgenden werden nun insbesondere jene Merkmale beschrieben, in denen sich die Handstücke 1 der Figuren 2 - 13 unterscheiden, bevorzugt die unterschiedlichen Temperaturmessvorrichtungen 4.

Die Handstücke 1 der Figur 2 - 4 weisen jeweils eine Temperaturmessvorrichtung 4 auf, die ausgebildet ist, die Temperatur des Handstücks 1 oder eines Teils des Handstücks 1 oder des durch die Wärmequellen 5, 6, 7 erwärmbaren Innenraums 11 berührungslos durch Detektion von elektromagnetischer Strahlung, insbesondere von Wärmestrahlung, zu messen.

Die Temperaturmessvorrichtungen 4 sind gemäß den Figuren 1 - 4 derart in den Handstücken 1 angeordnet und / oder ausgebildet, dass sie elektromagnetische Strahlung mehrerer in der Außenhülse 2 des Handstücks 1 angeordneter Wärmequellen (zum Beispiel der Lager 5, des Getriebes 6 und Teile der Werkzeuglösevorrichtung 7) empfangen, um insbesondere einen gemittelten Temperaturwert der mehreren Wärmequellen 5, 6, 7 zu bestimmen. Um die Wärmestrahlung der mehreren Wärmequellen 5, 6, 7 zu empfangen, ist zumindest eines der folgenden Merkmale verwirklicht: Die Temperaturmessvorrichtung 4 ist im Innenraum 11, 11' des Handstücks 1, beabstandet von den Wärmequellen 5, 6, 7 angeordnet; die Temperaturmessvorrichtung 4 ist zwischen den Wärmequellen 5, 6, 7 angeordnet; die Wärmequellen 5, 6, 7 umgeben die Temperaturmessvorrichtung 4; die elektromagnetische Strahlung emittierenden Oberflächen 8 (es ist in der Figur 2 beispielhaft nur eine derartige Oberfläche an einem Lager 5 dargestellt) der Wärmequellen 5, 6, 7 sind räumlich von einer die elektromagnetische Strahlung der Wärmequelle 5, 6, 7 empfangenden Oberfläche 8' der Temperaturmessvorrichtung 4 beabstandet; die Temperaturmessvorrichtung 4 weist mehrere elektromagnetische Strahlung der Wärmequelle 5, 6, 7 empfangende Oberflächen 8' auf, die insbesondere in unterschiedliche Richtungen weisen.

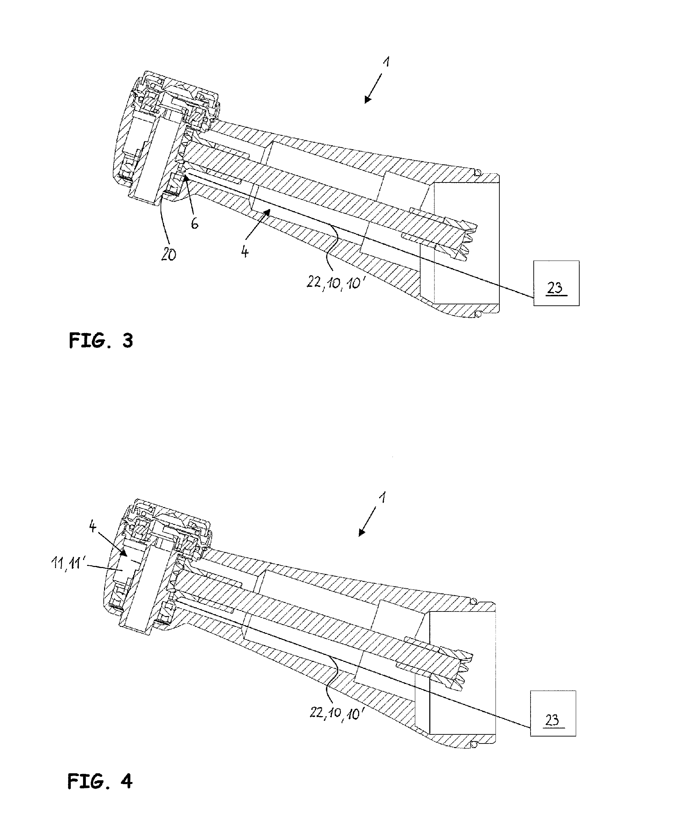

Vorzugsweise ist die Temperaturmessvorrichtung 4, insbesondere der Sensor der Temperaturmessvorrichtung 4, zwischen den beiden den Werkzeughalter 15 lagernden Lagern 5 und / oder in der Nähe der Kopfhülse 2', insbesondere an der Innenseite des sich zwischen dem Betätigungselement 21 und der Werkzeugöffnung 20 erstreckenden Mantels der Kopfhülse 2', und / oder in einem Raum zwischen der Kopfhülse 2' und dem Werkzeughalter 15 und / oder in einem Raum, der von der Kopfhülse 2, dem Werkzeughalter 15 und den beiden Lagern 5 begrenzt wird, angeordnet (siehe Figuren 2 und 4). Alternativ ist die Temperaturmessvorrichtung 4, insbesondere der Sensor der Temperaturmessvorrichtung 4, im Bereich der Werkzeuglösevorrichtung 7, insbesondere zwischen dem dem Betätigungselement 21 näher gelegenen Lager 5 und dem Betätigungselement 21 angeordnet. Alternativ ist die Temperaturmessvorrichtung 4, insbesondere der Sensor der Temperaturmessvorrichtung 4, in der Nähe jenes Lagers 5, das der Werkzeugöffnung 20 näher liegt, und / oder im Bereich des Getriebes 6 und / oder zwischen dem genannten Lager 5 und dem Getriebe 6 angeordnet (siehe Figur 3).

Die Temperaturmessvorrichtung 4 gemäß Figur 2 weist insbesondere ein Pyrometer oder einen Infrarotsensor 9 auf, die insbesondere ausgebildet sind, elektromagnetische Strahlung in einem Bereich von etwa 5 µm bis etwa 20 µm zu detektieren. Das Pyrometer oder der Infrarotsensor 9 sind bevorzugt als Halbleitersensor ausgebildet. Vorzugsweise umfasst das Pyrometer oder der Infrarotsensor 9 des Weiteren eine Vorrichtung zur Eigentemperaturkompensation, insbesondere einen Widerstand.

Die Temperaturmessvorrichtungen 4 gemäß den Figuren 3 und 4 weisen einen optischen Leiter 10 für elektromagnetische Strahlung auf, insbesondere einen Faserstab 10', vorzugsweise aus Quarzglas, dessen Streuung oder Brechungsindex, insbesondere im temperatursensitive Abschnitt, aufgrund von Temperaturänderungen variiert. In der Einheit 23 ist eine elektromagnetische Strahlungsquelle vorgesehen, welche Strahlung in den optischen Leiter abgibt. Diese Strahlung wird durch den optischen Leiter geleitet, in Abhängigkeit der Temperatur des Handstücks 1 oder des Innenraums 11 und damit in Abhängigkeit der Streuung oder des Brechungsindexes des optischen Leiters 10 gestreut bzw. gebrochen und kehrt anschließend zur Einheit 23 zurück. Die Einheit 23 ermittelt auf Basis der Veränderungen der empfangenen Strahlung in Bezug auf die von der Strahlungsquelle emittierte Strahlung die Temperatur (faseroptische Temperaturmessung oder distributed temperature sensing).

Wie im Vorstehenden bereits beschrieben, ist das Ende des optischen Leiters 10 oder der temperatursensitive Abschnitt des optischen Leiters 10 entweder im Bereich des der Werkzeugöffnung 20 näher gelegenen Lagers 5 (siehe Figur 3) oder in einem Raum zwischen der Kopfhülse 2' und dem Werkzeughalter 15 angeordnet (siehe Figur 4).

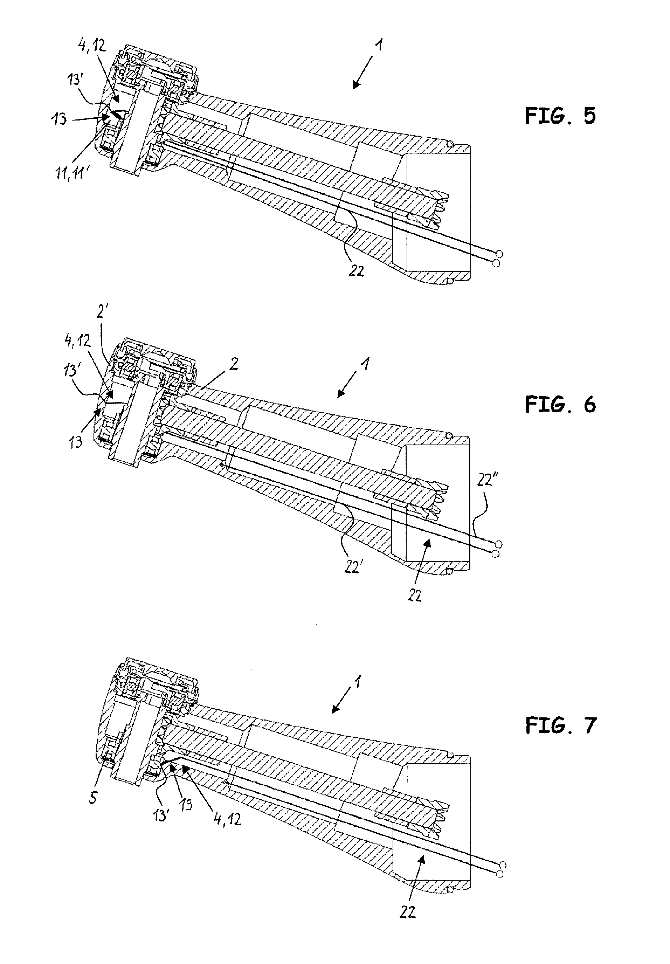

Die Figuren 5 - 10 zeigen unterschiedliche Ausführungsbeispiele eines Handstücks 1 mit einer elektrischen Temperaturmessvorrichtung 12, die insbesondere zumindest ein Thermoelement 13 oder eine Thermoelementverbindung 13' aufweist, welche ausgebildet sind, zur Messung der Temperatur ein elektrisches Thermospannungssignal zur Verfügung zu stellen. Das Thermoelement 13 oder die Thermoelementverbindung 13' weisen zwei unterschiedliche Materialien auf, insbesondere zwei unterschiedliche Metalle, die an einem Punkt oder Abschnitt miteinander verbunden sind, insbesondere verschweißt. Eine elektrische Leitung 22 leitet das Thermospannungssignal oder Messsignal des Thermoelements 13 zu der Einheit 23, die auf Basis des empfangenen Signals den Temperaturwert ermittelt.

Das Thermoelement 13 oder die Thermoelementverbindung 13' der Figur 5 ist ausgebildet und / oder angeordnet, die Temperatur des durch die zumindest eine Wärmequelle 5, 6, 7 erwärmbaren Innenraums 11 des Handstücks 1, bevorzugt die Temperatur des im Innenraum 11 oder Volumen 11' enthaltenen Fluids, zu messen. Alternativ ist das Thermoelement 13 oder die Thermoelementverbindung 13' der Figur 5 derart ausgebildet und / oder angeordnet, dass sie durch die Messung der Temperatur des Innenraums 11, 11' einen gemittelten Temperaturwert oder Durchschnittstemperaturwert ermittelt, insbesondere einen gemittelten Temperaturwert oder Durchschnittstemperaturwert mehrerer Bauteile und / oder Wärmequellen 5, 6, 7 des Handstücks 1, welche Wärme in den Innenraum 11, 11' oder an das darin enthaltene Fluid abgeben.

Um die Temperatur des Innenraums 11, 11' ermitteln zu können, ist zumindest eines der folgenden Merkmale verwirklicht: Das Thermoelement 13 oder die Thermoelementverbindung 13' ist im Innenraum 11, 11' des Handstücks 1, beabstandet von den Wärmequellen 5, 6, 7 angeordnet; das Thermoelement 13 oder die Thermoelementverbindung 13' ist zwischen den Wärmequellen 5, 6, 7 angeordnet; die Wärmequellen 5, 6, 7 umgeben das Thermoelement 13 oder die Thermoelementverbindung 13'; die Wärme emittierenden Oberflächen 8 der Wärmequellen 5, 6, 7 sind räumlich von dem Thermoelement 13 oder der Thermoelementverbindung 13' beabstandet.

Die Figur 5 zeigt konkret ein Thermoelement 13, das zwischen den beiden den Werkzeughalter 15 lagernden Lagern 5 und / oder in der Nähe der Kopfhülse 2', insbesondere an der Innenseite des sich zwischen dem Betätigungselement 21 und der Werkzeugöffnung 20 erstreckenden Mantels der Kopfhülse 2', und / oder in einem Raum zwischen der Kopfhülse 2' und dem Werkzeughalter 15 und / oder in einem Raum, der von der Kopfhülse 2, dem Werkzeughalter 15 und den beiden Lagern 5 begrenzt wird, angeordnet ist.

Alterativ sind in den Figuren 6 und 7 zwei Ausführungsbeispiele dargestellt, bei denen das Thermoelement 13 oder die Thermoelementverbindung 13' jeweils an einem Bauteil des Handstücks 1, nämlich der Außenhülse 2 (siehe Figur 5) oder einem Lager 5 (siehe Figur 6) vorgesehen ist, so dass das Thermoelement 13 oder die Thermoelementverbindung 13' vorzugsweise die Temperatur dieses Bauteils ermittelt. Bevorzugt weist das Bauteil 2, 5, an dem das Thermoelement 13 oder die Thermoelementverbindung 13' vorgesehen ist, ein Material auf, welches Teil des Thermoelements 13 oder der Thermoelementverbindung 13' ist.

Ein Vorteil der in den Figuren 6 und 7 dargestellten Ausführungsbeispiele liegt insbesondere darin, dass aufgrund der Verwendung eines Materials der Außenhülse 2, 2' als Teil des Thermoelements 13 oder der Thermoelementverbindung 13' oder aufgrund der elektrischen Verbindung der Außenhülse 2, 2' mit dem Thermoelement 13 oder der Thermoelementverbindung 13' oder aufgrund der elektrischen Verbindung der Außenhülse 2, 2' mit dem Bauteil 2, 5, dessen Material Teil des Thermoelements 13 oder der Thermoelementverbindung 13' ist, zumindest ein elektrischer Leiter 22' der elektrischen Leitung 22, die das Thermospannungssignal zur Einheit 23 leitet, das Thermospannungssignal nicht direkt an dem Thermoelement 13 oder der Thermoelementverbindung 13' abnehmen muss, sondern dass das Thermospannungssignal an (einer beliebigen Stelle) der Außenhülse 2, 2' abnehmbar ist.

In anderen Worten umfasst die Temperaturvorrichtung 4 gemäß den Figuren 6 und 7 ein Thermoelement 13 oder eine Thermoelementverbindung 13' und eine elektrische Leitung 22 mit zwei elektrischen Leitern 22', 22" zum Leiten des von dem Thermoelement 13 oder der Thermoelementverbindung 13' erzeugten Thermospannungssignals an die Auswerteeinheit 23, wobei das Thermoelement 13 oder die Thermoelementverbindung 13' an der Außenhülse 2, 2' oder an einem mit der Außenhülse 2, 2' (elektrisch) verbundenen Bauteil, zum Beispiel Lager 5, vorgesehen ist (um die Temperatur der Außenhülse 2, 2' oder des damit verbundenen Bauteils zu bestimmen), wobei die Außenhülse 2, 2' oder das Bauteil ein Material aufweist, welches Teil des Thermoelements 13 oder der Thermoelementverbindung 13' ist und wobei einer der beiden Leiter 22', 22" die Außenhülse 2, 2' des Handstücks 1 (elektrisch) kontaktiert, insbesondere eine beliebige Stelle der Außenhülse 2, 2' kontaktiert, vorzugsweise eine von dem Thermoelement 13 oder der Thermoelementverbindung 13' beabstandete Stelle der Außenhülse 2, 2', zum Beispiel den Griffteil 17 der Außenhülse 2 oder einen an den Kopfteil 16 anschließenden Abschnitt der Außenhülse 2 oder die Kupplungs- oder Anschlussvorrichtung 18 oder einen zwischen dem Thermoelement 13 oder der Thermoelementverbindung 13' und der Kupplungs- oder Anschlussvorrichtung 18 angeordnete Stelle der Außenhülse 2.

Alternativ ist es selbstverständlich in entsprechender Weise möglich, dass einer der beiden Leiter 22', 22" das Bauteil, dessen Material Teil des Thermoelements 13 oder der Thermoelementverbindung 13' ist, zum Beispiel das Lager 5, (elektrisch) kontaktiert, insbesondere eine beliebige Stelle des Bauteils kontaktiert. In diesem Fall ist klarerweise eine elektrische Verbindung des Bauteils zur Außenhülse 2, 2' nicht notwendig.

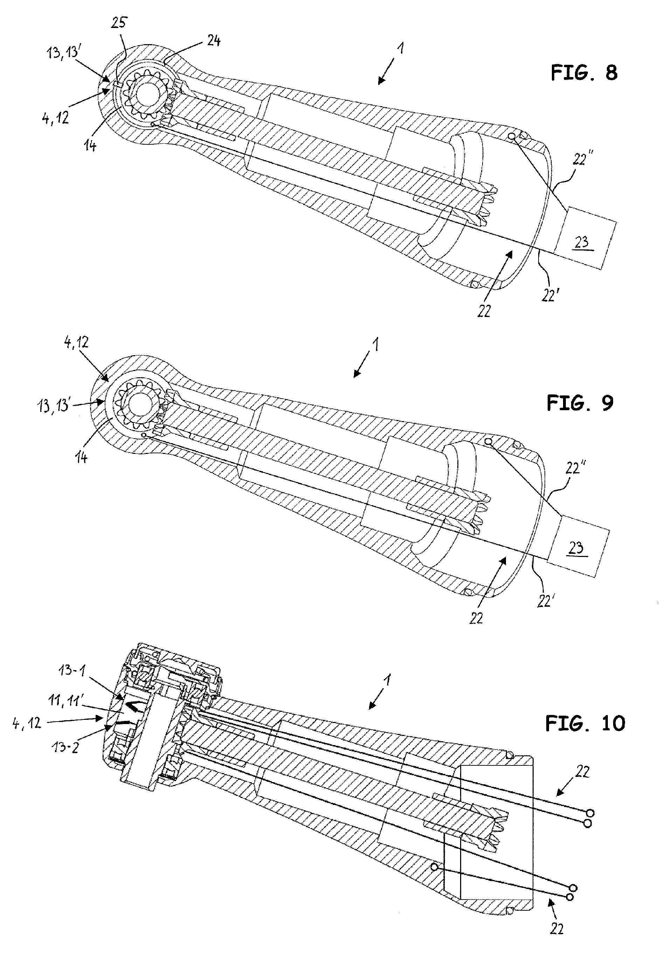

Die Figuren 8 und 9 zeigen zwei weitere Ausführungsbeispiele eines Handstücks 1 mit einem Thermoelement 13 oder einer Thermoelementverbindung 13', wobei bei beiden Ausführungsbeispielen ein, insbesondere metallischer, Kontaktring 14 vorgesehen ist, der Teil des Thermoelements 13 ist oder der einen Teil der Thermoelementverbindung 13' bildet.

In der Figur 8 ist der Kontaktring 14 durch eine elektrische Isolierung 24, zum Beispiel einer ringförmigen Isolierung, vom Handstück getrennt. Die elektrische Isolierung 24 ist insbesondere zwischen der Außenhülse 2, 2' des Handstücks 1 und dem Kontaktring 14 vorgesehen. An einer (räumlich begrenzten, schmalen oder punktförmigen) Kontaktstelle oder einem Kontaktpunkt 25 ist die Isolierung 24 unterbrochen oder weist einen Durchbruch auf, so dass ein direkter elektrischer Kontakt zwischen dem Kontaktring 14, der ein erstes Material aufweist, und der Außenhülse 2, 2' des Handstücks 1, die ein zweites, unterschiedliches Material aufweist, gebildet ist. Dieser die elektrische Isolierung 24 durchsetzende Kontaktpunkt 25 oder die beiden Materialien der Außenhülse 2, 2' und des Kontaktrings 14 bilden somit das Thermoelement 13 oder die Thermoelementverbindung 13' zur Messung der Temperatur. Das von dem Thermoelement 13 oder der Thermoelementverbindung 13' zur Verfügung gestellte Thermospannungssignal repräsentiert somit einen Temperaturwert an dem Punktkontakt zwischen der Außenhülse 2, 2' und dem Kontaktring 14. Vorzugsweise ist der Kontaktring 14 im Bereich des Betätigungselements 21 zum Betätigen der Werkzeuglösevorrichtung 7 vorgesehen, insbesondere zwischen dem Betätigungselement 21 und dem Lager 5, das dem Betätigungselement 21 näher liegt.

Das Handstück 1 der Figur 9 weist im Wesentlichen denselben Aufbau wie das Handstück 1 der Figur 8 auf, wobei jedoch keine elektrische Isolierung 24 zwischen der Außenhülse 2, 2' und dem Kontaktring 14 vorgesehen ist, so dass hier - anstelle des Punktkontakts der Figur 8 - zwischen der Außenhülse 2, 2' und dem Kontaktring 14 bzw. deren unterschiedlichen Materialien ein elektrischer Flächenkontakt vorliegt, der die Thermoelementverbindung 13' bildet. Der Flächenkontakt umfasst beispielhaft einen Winkel von zumindest 5° der Umfangsfläche des Kontaktrings 14, bevorzugt von zumindest 90°, insbesondere von mehr als 180°. Die von dem Thermoelement 13 oder der Thermoelementverbindung 13' zur Verfügung gestellte Thermospannung repräsentiert somit einen gemittelten Temperaturwert oder einen Durchschnittstemperaturwert an dem oder über den Flächenkontakt zwischen der Außenhülse 2, 2' und dem Kontaktring 14.

In den Handstücken 1 der Figuren 8 und 9 ist ebenfalls jeweils eine elektrische Leitung 22 zur Leitung des Thermospannungssignals vorgesehen, wobei einer der beiden Leiter 22' den Kontaktring 14 und der andere der beiden Leiter 22" die Außenhülse 2, 2' kontaktiert. Wie unter Bezug auf die Figuren 6 und 7 erläutert, kann der Leiter 22" die Außenhülse 2, 2' wiederum an einer beliebigen Stelle kontaktieren, vorzugsweise eine von dem Thermoelement 13 oder der Thermoelementverbindung 13' oder dem Kontaktring 14 beabstandete Stelle der Außenhülse 2, 2', zum Beispiel den Griffteil 17 der Außenhülse 2 oder einen an den Kopfteil 16 anschließenden Abschnitt der Außenhülse 2 oder die Kupplungs- oder Anschlussvorrichtung 18 oder einen zwischen dem Thermoelement 13 oder der Thermoelementverbindung 13' und der Kupplungs- oder Anschlussvorrichtung 18 angeordnete Stelle der Außenhülse 2.

Selbstverständlich ist es auch möglich, in einem Handstück 1 mehrere Thermoelemente 13 oder Thermoelementverbindungen 13' vorzusehen, so wie dies beispielhaft in der Figur 10 dargestellt ist. Das Handstück 1 der Figur 10 weist ein erstes Thermoelement 13-1 auf, das frei, ohne direkten Kontakt zu einem Bauteil des Handstücks 1 im Innenraum 11, 11' vorgesehen ist, um insbesondere die Temperatur des im Innenraum 11, 11' enthaltenen Fluids zu messen, so wie es im Vorstehenden für das Handstück 1 der Figur 5 beschrieben ist. Demgemäß sind alle in Zusammenhang mit dem Handstück 1 oder dem Thermoelement 13 der Figur 5 beschriebenen Merkmale in entsprechender Weise auf das Handstück 1 der Figur 10 oder das Thermoelement 13-1 anwendbar oder übertragbar.

Das Handstück 1 der Figur 10 weist des Weiteren ein zweites Thermoelement 13-2 auf, das an einem Bauteil des Handstücks 1 vorgesehen ist. Insbesondere weist das Bauteil ein Material auf, das Teil des Thermoelements 13-2 ist. Vorzugsweise ist das Bauteil durch die Außenhülse 2, 2' gebildet, so wie es im Vorstehenden für das Handstück 1 der Figur 6 beschrieben ist. Demgemäß sind alle in Zusammenhang mit dem Handstück 1 oder dem Thermoelement 13 der Figur 6 beschriebenen Merkmale in entsprechender Weise auf das Handstück 1 der Figur 10 oder das Thermoelement 13-2 anwendbar oder übertragbar.

Von jedem Thermoelement 13-1, 13-2 des Handstücks 1 führt eine elektrische Leitung 22 zu der Einheit 23, welche die Thermospannungssignale jedes Thermoelements 13-1, 13-2 empfängt und verarbeitet.

Weist ein Handstück 1 mehrere Thermoelement 13 oder der Thermoelementverbindung 13' auf, so sind selbstverständlich ihre Anzahl, als auch ihre Position im Handstück 1 oder ihre Bauweise je nach Anforderungen oder Wunsch, wo die Temperatur gemessen werden soll, frei wählbar. So können zum Beispiel in einem Handstück 1 mehrere freie Thermoelement 13-1 ohne direkten Kontakt zu einem Bauteil des Handstücks 1 und / oder mehrere an einem Bauteil und / oder an mehreren unterschiedlichen Bauteilen des Handstücks 1 angeordnete Thermoelement 13-2 vorgesehen sein.

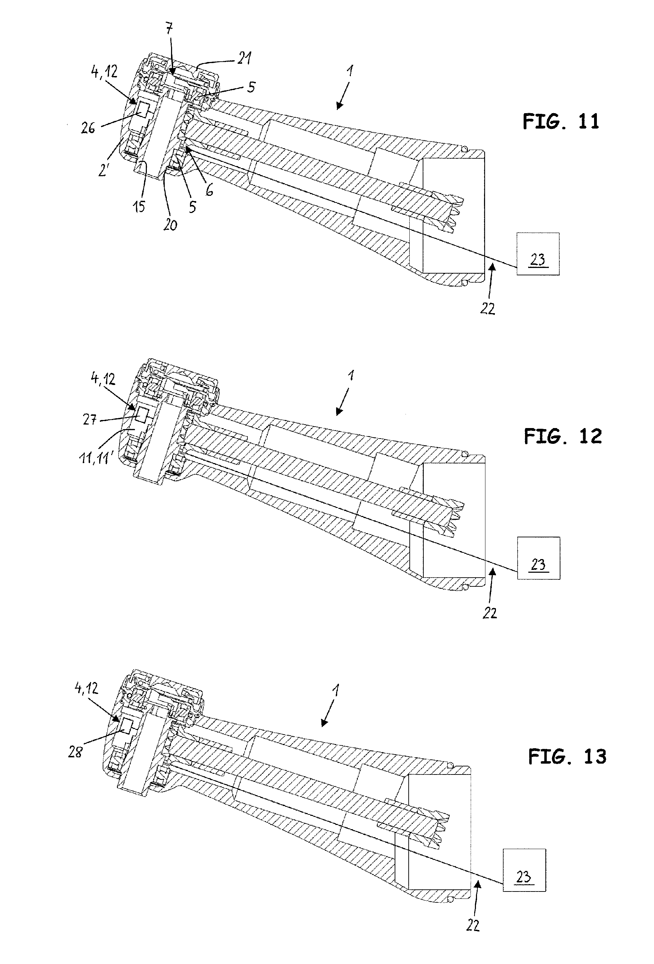

Die Figuren 11 - 13 zeigen Handstücke 1 mit weiteren elektrischen Temperaturmessvorrichtungen 12, wobei jede dieser Temperaturmessvorrichtungen 12 ein Material umfasst, dessen elektrischer Widerstand oder dynamischer Widerstand in Abhängigkeit der Temperatur variabel ist.

Die Temperaturmessvorrichtungen 4, 12 der Figuren 11 - 13 sind vorzugsweise derart in den Handstücken 1 angeordnet und / oder ausgebildet, dass sie Wärme oder Wärmestrahlung mehrerer in der Außenhülse 2 des Handstücks 1 angeordneter Wärmequellen (zum Beispiel der Lager 5, des Getriebes 6 und Teile der Werkzeuglösevorrichtung 7) empfangen, um insbesondere einen gemittelten Temperaturwert der mehreren Wärmequellen 5, 6, 7 zu bestimmen. Um die Wärme oder Wärmestrahlung der mehreren Wärmequellen 5, 6, 7 zu empfangen, ist zumindest eines der folgenden Merkmale verwirklicht: Die Temperaturmessvorrichtung 4, 12 ist im Innenraum 11, 11' des Handstücks 1, beabstandet von den Wärmequellen 5, 6, 7 angeordnet; die Temperaturmessvorrichtung 4, 12 ist zwischen den Wärmequellen 5, 6, 7 angeordnet; die Wärmequellen 5, 6, 7 umgeben die Temperaturmessvorrichtung 4, 12; die Wärme emittierenden Oberflächen der Wärmequellen 5, 6, 7 sind räumlich von einer die Wärme der Wärmequelle 5, 6, 7 empfangenden Oberfläche der Temperaturmessvorrichtung 4, 12 beabstandet; die Temperaturmessvorrichtung 4, 12 weist mehrere Wärme der Wärmequelle 5, 6, 7 empfangende Oberflächen 8' auf, die insbesondere in unterschiedliche Richtungen weisen.

Alternativ sind die Temperaturmessvorrichtungen 4, 12 der Figuren 11 - 13 ausgebildet und / oder angeordnet, die Temperatur des durch die zumindest eine Wärmequelle 5, 6, 7 erwärmbaren Innenraums 11 des Handstücks 1, bevorzugt die Temperatur des im Innenraum 11 oder Volumen 11' enthaltenen Fluids, zu messen.

Vorzugsweise ist die Temperaturmessvorrichtung 4, 12, insbesondere der Sensor der Temperaturmessvorrichtung 4, 12, zwischen den beiden den Werkzeughalter 15 lagernden Lagern 5 und / oder in der Nähe der Kopfhülse 2', insbesondere an der Innenseite des sich zwischen dem Betätigungselement 21 und der Werkzeugöffnung 20 erstreckenden Mantels der Kopfhülse 2', und / oder in einem Raum zwischen der Kopfhülse 2' und dem Werkzeughalter 15 und / oder in einem Raum, der von der Kopfhülse 2, dem Werkzeughalter 15 und den beiden Lagern 5 begrenzt wird, angeordnet (siehe Figuren 2 und 4). Alternativ ist die Temperaturmessvorrichtung 4, 12, insbesondere der Sensor der Temperaturmessvorrichtung 4, 12, im Bereich der Werkzeuglösevorrichtung 7, insbesondere zwischen dem dem Betätigungselement 21 näher gelegenen Lager 5 und dem Betätigungselement 21 angeordnet. Alternativ ist die Temperaturmessvorrichtung 4, 12, insbesondere der Sensor der Temperaturmessvorrichtung 4, 12, in der Nähe jenes Lagers 5, das der Werkzeugöffnung 20 näher liegt, angeordnet.

Die Temperaturmessvorrichtung 4, 12 des Handstücks 1 der Figur 11 weist einen Messwiderstand 26 auf, der vorzugsweise aus einem Edelmetall besteht, dessen elektrischer Widerstand (ohmsche Widerstand) in Abhängigkeit der Temperatur, insbesondere der Temperatur des den Widerstand 26 umgebenden Fluids des Innenraums 11, 11', variabel ist. Eine in der Einheit 23 vorgesehene Strom- oder Spannungsquelle bildet mit der elektrischen Leitung 22 und dem Messwiderstand 26 einen elektrischen Stromkreis. Die Veränderung des von der Strom- oder Spannungsquelle abgegebenen, über den Messwiderstand 26 geleiteten elektrischen Messsignals, insbesondere der Spannungsabfall eines als Messsignal dienenden konstanten Messstroms, wird von einem Schaltkreis der Einheit 23 detektiert und zur Bestimmung der Temperatur verwendet. Vorzugsweise sind in der Einheit weitere Elemente zur Verarbeitung des Messsignals vorhanden, zum Beispiel Analog-Digital-Wandler, Signalverstärker oder Filter.

Die Temperaturmessvorrichtung 4, 12 des Handstücks 1 der Figur 12 weist einen Halbleitersensor 27 auf, vorzugsweise eine Halbleiterdiode, insbesondere eine dotierte Siliziumdiode, dessen dynamischer Widerstand (Schleusenspannung an einem p-n-Übergang oder einem Metall-Halbleiter-Übergang des Halbleitersensors 27) in Abhängigkeit der Temperatur, insbesondere der Temperatur des den Halbleitersensor 27 umgebenden Fluids des Innenraums 11, 11', variabel ist. Eine in der Einheit 23 vorgesehene Strom- oder Spannungsquelle bildet mit der elektrischen Leitung 22 und dem Halbleitersensor 27 einen elektrischen Stromkreis. Die Veränderung des von der Strom-oder Spannungsquelle abgegebenen, über den Halbleitersensor 27 geleiteten elektrischen Messsignals, insbesondere der Spannungsabfall eines als Messsignal dienenden konstanten Messstroms, wird von einem Schaltkreis der Einheit 23 detektiert und zur Bestimmung der Temperatur verwendet. Vorzugsweise sind in der Einheit weitere Elemente zur Verarbeitung des Messsignals vorhanden, zum Beispiel Analog-Digital-Wandler, Signalverstärker oder Filter.

Die Temperaturmessvorrichtung 4, 12 des Handstücks 1 der Figur 12 weist einen metallischen Widerstand 28 auf, wobei die Spannung des thermischen Widerstandsrauschens (d.h. die Dichteschwankungen der Transportelektronen des Widerstands 28 aufgrund ihrer thermischen Bewegung) in Abhängigkeit der Temperatur, insbesondere der Temperatur des den Widerstand 28 umgebenden Fluids des Innenraums 11, 11', variabel ist. Eine in der Einheit 23 vorgesehene Strom-oder Spannungsquelle bildet mit der elektrischen Leitung 22 und dem Widerstand 28 einen elektrischen Stromkreis. Die Veränderung des von der Strom- oder Spannungsquelle abgegebenen, über den Widerstand 28 geleiteten elektrischen Messsignals, insbesondere die Veränderung der elektrischen Spannung, wird von einem Schaltkreis der Einheit 23 detektiert und zur Bestimmung der Temperatur verwendet. Vorzugsweise sind in der Einheit weitere Elemente zur Verarbeitung des Messsignals vorhanden, zum Beispiel Analog-Digital-Wandler, Signalverstärker oder Filter.

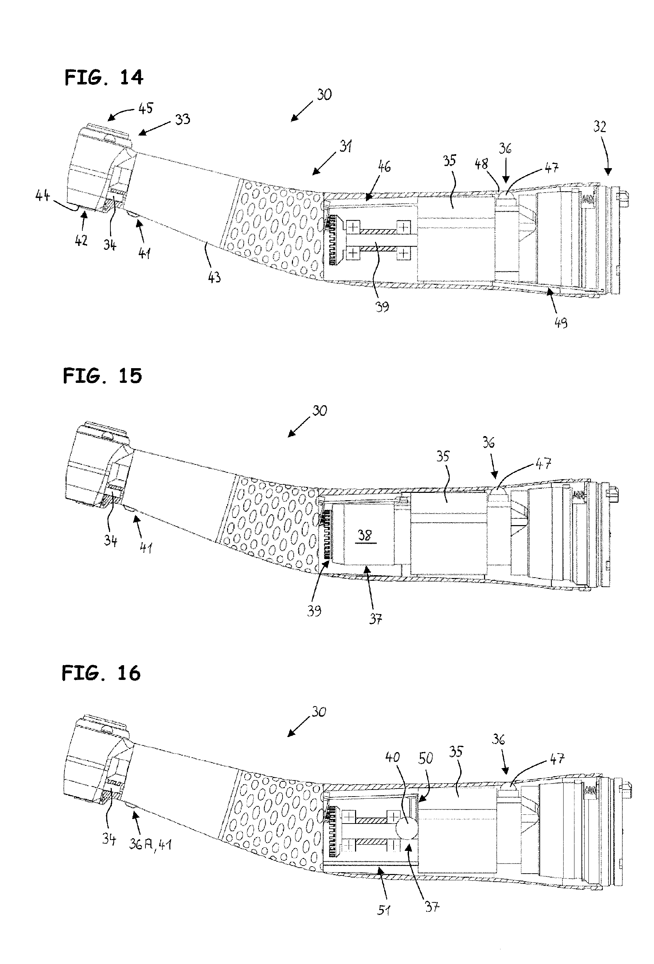

Die Figuren 14 - 16 zeigen Hand- oder Winkelstücke 30 mit einer von einer Steuer-, Regel-und / oder Versorgungseinheit größtenteils oder vollständig unabhängigen Temperaturmessung. Zunächst werden die für die Handstücke 30 der Figuren 14 - 16 gemeinsamen Merkmale und Bauteile beschrieben:

Das Handstück 30 oder dessen Außenhülse 43 umfasst ein Griffteil 31 zum Halten des Handstücks 30, insbesondere während des Betriebs des Handstücks 30, ein damit verbundenes oder verbindbares Kupplungsteil 32 zur Verbindung des Handstücks 30 mit zumindest einer Medienquelle und / oder einer Energiequelle, insbesondere zur Verbindung mit einer Steuer-, Regel- und / oder Versorgungseinheit, und ein mit dem Griffteil 31 verbundenes oder verbindbares Kopfteil 33 mit einer in Bewegung versetzbaren Haltevorrichtung 42 für ein Werkzeug. Die Haltevorrichtung 42 umfasst zum Beispiel eine kraftschlüssige Spannzange oder eine formschlüssige Halteeinrichtung für das Werkzeug. Durch eine im Kopfteil 33 oder in dessen Außenhülse vorgesehene Werkzeugaufnahmeöffnung 44 kann das Werkzeug in die Haltevorrichtung 42 eingebracht oder daraus gelöst werden. Am Kopfteil 33 ist des Weiteren eine Lösevorrichtung 45 vorgesehen, mit der das Werkzeug aus der Haltevorrichtung 42 lösbar ist.

Am Kopfteil 33 oder daran anschließend ist eine Beleuchtungsvorrichtung 41 vorgesehen, die Licht in Richtung des Werkzeugs und / oder der Behandlungsstelle abstrahlt. Die Beleuchtungsvorrichtung 41 weist zum Beispiel ein optisches, Licht emittierendes Halbleiterelement oder eine Glühbirne und / oder einen optischen Lichtleiter, zum Beispiel eine oder mehrere optischen Fasern oder einen Glasstab auf.

Die in den Figuren 14 - 16 dargestellten Handstücke 30 sind mechanisch betreibbar, vorzugsweise motorisch, insbesondere mittels eines Elektromotors, und weisen zumindest eine Antriebswelle 39 auf, bevorzugt mit einem Zahnrad, welche zum Übertragen einer Antriebsbewegung auf die Haltevorrichtung 42 ausgebildet ist. Selbstverständlich ist es jedoch genauso möglich, die Haltevorrichtung 42 mittels eines Antriebsfluids, insbesondere mittels Druckluft, anzutreiben und in Bewegung zu versetzen, insbesondere mit einem mit der Haltevorrichtung 42 verbundenen, mit dem Antriebsfluid beaufschlagbaren Laufrad oder Rotor.

Aus dem Vorstehenden ist zu erkennen, dass zumindest ein bewegbares Bauteil in dem Handstück 30 angeordnet ist, zum Beispiel die Antriebswelle 39, die Haltevorrichtung 42, das Laufrad oder der Rotor oder Lager, vorzugsweise Wälz- oder Kugellager, welche insbesondere die vorgenannten Bauteile im Handstück 30 lagern. Aufgrund der Bewegung dieses zumindest einen bewegbar angeordneten Bauteils und / oder aufgrund einer Relativbewegung und somit Reibung zwischen diesem zumindest einen bewegbaren Bauteil und der Außenhülse 43 oder einem drehfest in der Außenhülse 43 aufgenommenen Bauteil kann es zu einer erheblichen Erwärmung des Handstücks 30 oder von Teilen davon kommen. Selbstverständlich kann auch eine andere Wärmequelle eine Erwärmung des Handstücks 30 bewirken, zum Beispiel eine mit elektrischer Energie betreibbare Vorrichtung, vorzugsweise ein Motor, eine Heizvorrichtung oder eine Beleuchtungsvorrichtung.

Um die Temperatur des Handstücks messen und / oder anzeigen und / oder überwachen und / oder beeinflussen zu können, ist am Handstück 30 eine Temperaturmessvorrichtung 34 vorgesehen. Die Temperaturmessvorrichtung 34, insbesondere zumindest ein Temperatursensor der Temperaturmessvorrichtung 34, kann an einem beliebigen Ort oder Teil 31, 32, 33 des Handstücks 30 vorgesehen sein, vorzugsweise in der Nähe zumindest einer Wärmequelle oder an einer Stelle des Handstücks 30, die häufig mit dem Anwender oder einem Patienten in Berührung kommt, insbesondere in dem Kopfteil 33 oder anschließend an das Kopfteil 33, in dem oftmals mehrere bewegbare und / oder relativ zueinander bewegbare Bauteil angeordnet sind, oder an zumindest einem Teil der Lösevorrichtung 45, insbesondere an oder nahe an deren Drucktaster oder Betätigungselement. Die Temperaturmessvorrichtung 34 oder deren Temperatursensor kann zum Beispiel ein Thermoelement oder einen Infrarotsensor umfassen.

Die Temperaturmessvorrichtung 34 ist ausgebildet, ein elektrisches Temperaturmesssignal bereit zu stellen, welches an eine elektrische Schaltvorrichtung 35 übertragbar ist. Die Übertragung des Temperaturmesssignals erfolgt vorzugsweise leitungsgebunden mittels einer elektrischen Leitung 46, welche die Temperaturmessvorrichtung 34 mit der Schaltvorrichtung 35 verbindet. Die Schaltvorrichtung 35, insbesondere in Form eines Mikrocontrollers, ist ausgebildet, das elektrische Temperaturmesssignal mit einem Vergleichswert, insbesondere mit einem Temperaturgrenzwert, zu vergleichen und eine Signalvorrichtung 36 zumindest zwischen einem ersten Signalzustand und einem zweiten Signalzustand, der sich vom ersten Signalzustand unterscheidet, zu schalten, wenn das Temperaturmesssignal den Vergleichswert oder Temperaturgrenzwert erreicht oder überschreitet oder unterschreitet. Der erste und zweite Signalzustand und / oder der Wechsel zwischen den beiden Signalzuständen sind derart ausgebildet, dass sie den Anwender auf das Erreichen, Über- oder Unterschreiten des Temperaturgrenzwerts aufmerksam machen.

Die vorzugsweise wiederum mittels elektrischer Leitungen mit der Schaltvorrichtung 35 verbundene Signalvorrichtung 36 umfasst gemäß den Figuren 14 - 16 eine Lichtquelle 47, insbesondere zumindest eine Leuchtdiode. Die Signalvorrichtung 36 kann jedoch alternativ oder zusätzlich einen anderen Signalgeber aufweisen, zum Beispiel ein in Schwingung versetzbares Element, das vom Anwender akustisch und / oder taktil wahrnehmbar ist. Die Signalvorrichtung 36 ist gemäß den Figuren 14 - 16 am Kupplungsteil 32 oder am Griffteil 31, nahe des Kupplungsteils 32 angeordnet, sie kann jedoch auch an einer beliebigen anderen Stelle des Handstücks 30 vorgesehen sein, insbesondere an einer von der Werkzeugaufnahmeöffnung 44 abgewandten Seite des Handstücks 30, die während der Behandlung dem Anwender zugewandt ist oder für den Anwender besonders gut sichtbar ist. In der Außenhülse 43 ist bevorzugt eine Öffnung 48 vorgesehen, durch welche die Signalvorrichtung 36 ihr Signal, insbesondere die Lichtquelle 47 ihr Licht, abgegeben kann. Vorzugsweise ist zumindest ein Teil der Signalvorrichtung 36, insbesondere der Lichtquelle 47 in der Öffnung 48 angeordnet.

Die Versorgung der Temperaturmessvorrichtung 34, der Schaltvorrichtung 35 und gegebenenfalls auch der Signalvorrichtung 36 mit elektrischer Energie ist bei den Handstücken 30 der Figuren 14 - 16 unterschiedlich:

Das Handstück 30 der Figur 14 ist über das Kupplungsteil 32 mit einer außerhalb des Handstücks 30 angeordneten Energiequelle verbindbar, wobei an dem Kupplungsteil 32 lösbar mit der Energiequelle verbindbare elektrische Kontakte vorgesehen sind. Eine im Handstück 30 angeordnete elektrische Leitung 49 verbindet die elektrischen Kontakte am Kupplungsteil 32 mit den im Vorstehenden genannten Verbrauchern 34, 35, 36. Vorzugsweise verbindet die elektrische Leitung 49 die Kontakte direkt mit der Schaltvorrichtung 35, die insbesondere mit einer Vorrichtung zum Anpassen des Stroms oder der Spannung versehen ist, um die anderen Verbraucher 34, 36 mit der jeweils benötigten Spannung und / oder dem benötigten Strom zu versorgen. Insbesondere ist die Schaltvorrichtung 35 ausgebildet, zumindest einen Teil der elektrischen Energie als Schaltsignal für die Signalvorrichtung 36 zu verwenden (letzteres trifft auch für die Handstücke der Figuren 15 und 16 zu).

Die Figuren 15, 16 zeigen zwei Ausführungsbeispiele von Handstücken 30, bei denen die Versorgung mit elektrischer Energie durch eine im oder am Handstück 30 vorgesehene Energiequelle 37 erfolgt. Gemäß der Figur 15 weist das Handstück 30 einen elektrodynamischen Wandler (Generator) 38 auf, der durch die Antriebswelle 39 betreibbar ist und, sobald die Welle 39, die mit einem Rotor des Generators 38 verbunden ist, in Drehung versetzt wird, elektrische Energie erzeugt. Der Aufbau eines derartigen Generators 38 ist aus dem Stand der Technik bekannt, so dass darauf nicht näher eingegangen wird. Zur Übertragung der vom Generator 38 erzeugten elektrischen Energie auf die Verbraucher 34, 35, 36 ist eine elektrische Leitung vorgesehen.

Alternativ ist in der Figur 16 ein Handstück 30 dargestellt, dessen im oder am Handstück 30 vorgesehene Energiequelle 37 durch einen Energiespeicher 40, insbesondere durch eine Batterie oder einen mehrfach aufladbaren Akkumulator, gebildet ist. Zur Übertragung der elektrischen Energie vom Energiespeicher 40 auf die Verbraucher 34, 35, 36 ist eine elektrische Leitung 50 vorgesehen.

In der Figur 16 ist zusätzlich eine weitere elektrische Leitung 51 dargestellt, welche die Beleuchtungsvorrichtung 41, die zum Beispiel ein optisches, Licht emittierendes Halbleiterelement oder eine Glühbirne umfasst, mit elektrischer Energie versorgt. Die Beleuchtungsvorrichtung 41 ist ausgebildet, Licht in Richtung des Werkzeugs und / oder der Behandlungsstelle abzustrahlen. Zusätzlich ist die Beleuchtungsvorrichtung 41 als Signalvorrichtung 36A ausgebildet, die von der Schaltvorrichtung 35 zumindest zwischen einem ersten Signalzustand und einem zweiten Signalzustand, der sich vom ersten Signalzustand unterscheidet, schaltbar ist, wenn das Temperaturmesssignal einen Grenzwert erreicht oder überschreitet oder unterschreitet. Vorzugsweise sind der erste Signalzustand durch die Abgabe von Licht und der zweite Signalzustand durch keine Abgabe von Licht durch die Beleuchtungsvorrichtung 41 definiert.

Die elektrische Leitung 51 verbindet die Schaltvorrichtung 35 mit der Beleuchtungsvorrichtung 41 und ermöglicht somit der Schaltvorrichtung 35 die Beleuchtungsvorrichtung 41 zwischen den beiden Signalzuständen zu schalten, zum Beispiel durch Unterbrechen und Öffnen der Versorgung der Beleuchtungsvorrichtung 41 mit elektrischer Energie. Die Beleuchtungsvorrichtung 41 ist entweder mit elektrischer Energie von einer außerhalb des Handstücks 30 angeordneten Energiequelle oder von einer im oder am Handstück 30 angeordneten Energiequelle 37 versorgbar.

Wenn die Beleuchtungsvorrichtung 41 als Signalvorrichtung 36A ausgebildet ist, ist es bevorzugt auch möglich, die Signalvorrichtung 36 wegzulassen, so dass die Beleuchtungsvorrichtung 41 die einzige durch die Schaltvorrichtung 35 schaltbare Signalvorrichtung zur Temperaturanzeige ist. Des Weiteren ist klarerweise die als Signalvorrichtung 36A ausgebildete Beleuchtungsvorrichtung 41 nicht auf das Ausführungsbeispiel der Figur 16 beschränkt, sondern ist in entsprechender Weise in die Handstücke 30 der Figuren 14, 15 implementierbar, vorzugsweise wiederum unter Weglassen der jeweiligen Signalvorrichtungen 36.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. So ist insbesondere die Position der Temperaturvorrichtung 4, 34 im Handstück 1, 30 variabel und nicht ausschließlich auf den Kopfteil 16, 33 beschränkt. Generell kann die Temperaturvorrichtung 4, 34 an beliebigen Stellen im Handstück 1, 30 angeordnet sein, insbesondere dort, wo Wärmequellen im Handstück 1, 30 positioniert sind oder wo eine Außenfläche des Handstücks 1, 30 besonders warm wird.

Des Weiteren sind alle Merkmale aller beschriebenen und dargestellten Ausführungsbeispiele miteinander kombinierbar. Insbesondere sind Temperatursensoren oder spezifische Methoden der Temperaturmessung, die unter Bezugnahme auf ein Ausführungsbeispiel beschrieben sind, auch in Handstücke anderer Ausführungsbeispiele implementierbar. Es ist zum Beispiel auch möglich, unterschiedliche Sensoren in einem Handstück 1, 30 vorzusehen, zum Beispiel einen Infrarotsensor 9 und ein Thermoelement 13. Die von den unterschiedlichen Sensoren abgegebenen Messsignale können entweder getrennt voneinander ausgewertet werden und / oder für eine gemeinsame, kombinierte Auswertung zusammengeführt werden, zum Beispiel zur Bildung eines Temperatur-Mittelwerts oder eines gewichteten Temperaturwerts, vorzugsweise durch eine Steuer-, Regel- und / oder Versorgungseinheit 23.

## Patentansprüche

1. Medizinisches, insbesondere dentales, Handstück (30), umfassend: Ein Griffteil (31), ein Kupplungsteil (32) zur Verbindung des Handstücks (30) mit zumindest einer Medienquelle und / oder einer Energiequelle, ein Kopfteil (33) mit einer in Bewegung versetzbaren Haltevorrichtung (42) für ein Werkzeug und eine Temperaturmessvorrichtung (34) zur Messung der Temperatur in dem Handstück (30) oder zumindest eines Teils des Handstücks (30), welche ausgebildet ist, ein elektrisches Temperaturmesssignal bereit zu stellen, **gekennzeichnet durch**
eine elektrische Schaltvorrichtung (35), die elektrisch mit der Temperaturmessvorrichtung (34) und mit einer Signalvorrichtung (36, 36A) verbunden ist, um das von der Temperaturmessvorrichtung (34) bereitgestellte Temperaturmesssignal zu empfangen und die Signalvorrichtung (36, 36A) zumindest zwischen einem ersten Signalzustand und einem zweiten Signalzustand, der sich vom ersten Signalzustand unterscheidet, zu schalten, wenn das Temperaturmesssignal einen Grenzwert erreicht oder überschreitet oder unterschreitet.

2. Medizinisches, insbesondere dentales, Handstück (30) nach Anspruch 1, **dadurch gekennzeichnet, dass**
die elektrische Schaltvorrichtung (35) und / oder die Temperaturmessvorrichtung (34) und / oder die Signalvorrichtung (36, 36A) zur Versorgung mit elektrischer Energie über das Kupplungsteil (32) mit einer außerhalb des Handstücks (30) angeordneten Energiequelle verbindbar ist / sind.

3. Medizinisches, insbesondere dentales, Handstück (30) nach Anspruch 1, **dadurch gekennzeichnet, dass**
die elektrische Schaltvorrichtung (35) und / oder die Temperaturmessvorrichtung (34) und / oder die Signalvorrichtung (36, 36A) zur Versorgung mit elektrischer Energie mit einer im oder am Handstück (30) vorgesehenen Energiequelle (37) verbunden ist / sind.

4. Medizinisches, insbesondere dentales, Handstück (30) nach Anspruch 3, **dadurch gekennzeichnet, dass**
die im oder am Handstück (30) vorgesehene Energiequelle (37) einen elektrodynamischen Wandler (38) aufweist, der durch eine Antriebswelle (39) des Handstücks (30) oder durch ein im Handstück (30) förderbares Fluid betreibbar ist.

5. Medizinisches, insbesondere dentales, Handstück (30) nach Anspruch 3, **dadurch gekennzeichnet, dass**
die im oder am Handstück (30) vorgesehene Energiequelle (37) einen Energiespeicher (40) umfasst, insbesondere eine Batterie oder einen wiederaufladbaren Akkumulator.

6. Medizinisches, insbesondere dentales, Handstück (30) nach einem der vorstehenden Ansprüche, **dadurch gekennzeichnet, dass**
der erste Signalzustand und der zweite Signalzustand ein visuell wahrnehmbares und / oder ein akustisch wahrnehmbares und / oder ein taktil wahrnehmbares Signal umfassen.

7. Medizinisches, insbesondere dentales, Handstück (30) nach einem der vorstehenden Ansprüche, **dadurch gekennzeichnet, dass**
die Signalvorrichtung (36, 36A) eine Lichtquelle (47), insbesondere ein optisches Halbleiterelement, aufweist.

8. Medizinisches, insbesondere dentales, Handstück (30) nach einem der vorstehenden Ansprüche, **dadurch gekennzeichnet, dass**
die Signalvorrichtung (36, 36A), insbesondere die Lichtquelle (47), ausgebildet ist, den ersten Signalzustand durch Anzeigen einer ersten Farbe und den zweiten Signalzustand durch Anzeigen einer zweiten Farbe, die sich von der ersten Farbe unterscheidet, darzustellen.

9. Medizinisches, insbesondere dentales, Handstück (30) nach einem der vorstehenden Ansprüche, **dadurch gekennzeichnet, dass**
die Signalvorrichtung (36, 36A), insbesondere die Lichtquelle (47), ausgebildet ist, den ersten Signalzustand durch Absenden von Licht vom Handstück (30) und den zweiten Signalzustand durch Nicht-Absenden von Licht vom Handstück (30) darzustellen.

10. Medizinisches, insbesondere dentales, Handstück (30) nach einem der vorstehenden Ansprüche, **dadurch gekennzeichnet, dass**
die Signalvorrichtung (36, 36A) eine Beleuchtungsvorrichtung (41) umfasst, die derart am Handstück (30) angeordnet ist, dass sie ein in die Haltevorrichtung (42) des Handstücks (30) einsetzbares Werkzeug und / oder die Behandlungsstelle beleuchtet.

11. Medizinisches, insbesondere dentales, Handstück (30) nach einem der vorstehenden Ansprüche, **dadurch gekennzeichnet, dass**
die Signalvorrichtung (36, 36A), insbesondere die Lichtquelle (47), am Griffteil (31) oder am Kupplungsteil (32) des Handstücks (30) vorgesehen ist.

12. Medizinisches, insbesondere dentales, Handstück (30) nach einem der vorstehenden Ansprüche, **dadurch gekennzeichnet, dass**
die Temperaturmessvorrichtung (34) im oder anschließend an das Kopfteil (33) des Handstücks (30) und / oder an oder nahe an einem bewegten Bauteil des Handstücks (30) angeordnet ist.

13. Medizinisches, insbesondere dentales, Handstück (30) nach einem der vorstehenden Ansprüche, **dadurch gekennzeichnet, dass**
die Temperaturmessvorrichtung (34) ausgebildet ist, die Temperatur des Handstücks (30) oder eines Teils des Handstücks (30) berührungslos durch Detektion von elektromagnetischer Strahlung, insbesondere von Wärmestrahlung, zu messen und / oder dass die Temperaturmessvorrichtung (34) ausgebildet ist, die Temperatur eines durch zumindest eine Wärmequelle erwärmbaren Innenraums des Handstücks (30) zu messen.

14. Medizinisches, insbesondere dentales, Handstück (30) nach einem der vorstehenden Ansprüche, **dadurch gekennzeichnet, dass**
das Handstück (30) zumindest zwei lösbar miteinander verbundene Teile aufweist, wobei die elektrische Schaltvorrichtung (35) und / oder die Temperaturmessvorrichtung (34) und / oder die Signalvorrichtung (36, 36A) und / oder die im oder am Handstück (30) vorgesehenen Energiequelle (37) in unterschiedlichen Teilen angeordnet sind.
